(19) Europäisches Patentamt
European Patent Office
Office européen des brevets

(11) EP 1 329 722 A1

(12) # EUROPEAN PATENT APPLICATION
published in accordance with Art. 158(3) EPC

(43) Date of publication:
**23.07.2003 Bulletin 2003/30**

(21) Application number: **01972552.2**

(22) Date of filing: **27.09.2001**

(51) Int Cl.7: **G01N 33/72**, G01N 21/78

(86) International application number:
**PCT/JP01/08484**

(87) International publication number:
**WO 02/027331 (04.04.2002 Gazette 2002/12)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **28.09.2000 JP 2000296538**

(71) Applicant: **ARKRAY, Inc.**
**Kyoto-shi, Kyoto 601-8045 (JP)**

(72) Inventors:
- **HIRAI, Kaoru, c/o ARKRAY, INC.**
**Kyoto-shi, Kyoto 601-8045 (JP)**
- **KOMORI, Tsuguki, c/o ARKRAY, INC.**
**Kyoto-shi, Kyoto 601-8045 (JP)**

(74) Representative: **VOSSIUS & PARTNER**
**Siebertstrasse 4**
**81675 München (DE)**

(54) **ASSAY METHOD WITH THE USE OF REDOX REACTION**

(57) A method of determining an analyte in a sample utilizing a redox reaction is provided, by which a highly reliable determined value can be obtained. In this method, a tetrazolium compound is added to a sample in the presence of a surfactant prior to a redox reaction so as to eliminate an effect of hemoglobin and a hemoglobin degradation product as reducing substances in the sample. Thereafter, a reducing or oxidizing substance derived from an analyte is produced and the amount thereof is determined by the redox reaction. Then, the amount of the analyte in the sample is determined based on the amount of the reducing or oxidizing substance thus determined. According to this method, cloudiness due to the coexistence with the hemoglobin can be prevented by the surfactant and thus an increase in the absorbance due to the cloudiness can be inhibited as shown in FIG. 1. As the surfactant, polyoxyethylene ether and the like can be used.

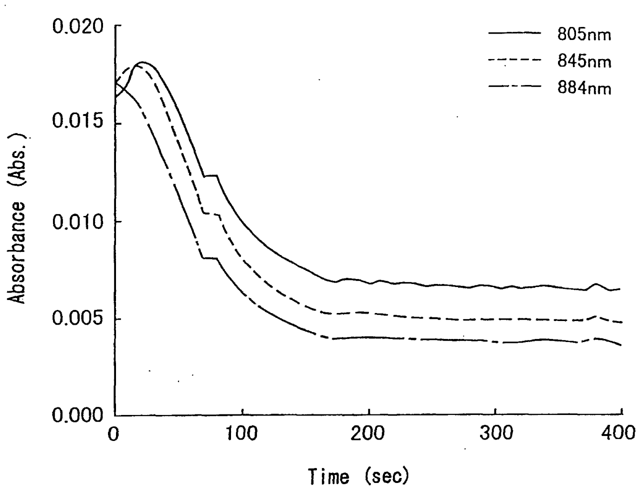


FIG. 1

EP 1 329 722 A1

## Description

### Technical Field

The present invention relates to a method of determining an analyte in a sample utilizing a redox reaction.

### Background Art

Conventionally, determination of an analyte in a sample utilizing a redox reaction has been employed widely. For example, such a determination is applied for determining a glycated protein in biochemical analysis, clinical tests, and the like.

For example, the glycated protein in blood, especially glycated hemoglobin in red blood cells, has served as an important index for the diagnosis, treatment, etc. of diabetes because it reflects previous blood glucose levels in vivo. The glycated protein in red blood cells is determined utilizing a redox reaction, for example, in the following manner.

First, red blood cells are hemolyzed to prepare a sample. Then, this hemolysate sample is treated with a fructosyl amino acid oxidase (hereinafter, referred to as "FAOD") so that the FAOD acts on a glycation site of a glycated protein, thereby causing hydrogen peroxide to be generated. The amount of the hydrogen peroxide thus generated corresponds to the amount of the glycated protein. Subsequently, a peroxidase (hereinafter, referred to as "POD") and a reducing agent are added to the sample so that the POD catalyzes a redox reaction between the hydrogen peroxide and the reducing agent. In the case where a color-developing substrate that develops color by oxidation is used as the reducing agent, the amount of the hydrogen peroxide can be determined by measuring the color development, and the amount of the glycated protein in the red blood cells can be known from the amount of the hydrogen peroxide thus determined.

However, in general, blood contains various reducing substances such as ascorbic acid (AsA) and bilirubin, and red blood cells contain various reducing substances such as glutathione (GSH). These reducing substances may reduce the hydrogen peroxide generated, may inhibit the redox reaction, and may reduce the oxidized color-developing substrate again so that the color developed by the substrate is faded, for example. Therefore, it is difficult to determine the amount of the glycated protein in the red blood cell accurately.

Besides, since the concentrations of the reducing substances contained in samples vary from one sample to another, the accuracy of determination is not sufficient.

Examples of a method for solving these problems include adding various oxidizing agents to the sample. For example, JP 56(1981)-151358 A discloses a method in which a halogen oxide such as iodic acid and periodic acid is used as an oxidizing agent. Further, JP 57(1982)-13357 A, JP 57(1982)-161650 A, JP 59(1984)-193354 A, JP 62 (1987)-169053 A, and JP 3(1991)-30697 A disclose a method in which a metal complex such as cobalt, iron, and cerium is used as an oxidizing agent.

### Disclosure of Invention

However, depending on the sample to be used, even the thus-modified conventional methods may fail to improve the accuracy of the determination sufficiently. Further, since the glycated protein in blood has served as an important index for the diagnosis, treatment, etc. of diabetes as described above, still further improvement in the accuracy of the determination is desired in the method of determining the glycated protein utilizing a redox reaction.

Therefore, it is an object of the present invention to provide a method of determining an analyte in a sample utilizing a redox reaction, by which a highly reliable determined value can be obtained.

In order to achieve the above object, a method of determining an analyte in a sample utilizing a redox reaction according to the present invention includes: adding a tetrazolium compound to a sample in the presence of a surfactant prior to a redox reaction, thereby eliminating an effect of a reducing substance in the sample; determining an amount of a reducing or oxidizing substance derived from an analyte utilizing the redox reaction; and determining an amount of the analyte based on the amount of the reducing or oxidizing substance thus determined. The tetrazolium compound is a compound having a tetrazole ring structure. A "reducing or oxidizing substance derived from an analyte" as used in the present invention refers to the analyte itself or a reducing/oxidizing substance contained therein, or a reducing/oxidizing substance that is caused to be produced from the analyte by an oxidoreductase or the like.

The inventors of the present invention have found the following. According to the conventional methods as described above, the effect of a high-molecular-weight reducing substance such as proteins and the like cannot be eliminated while the effect of a low-molecular-weight reducing substance such as the above-mentioned GSH and AsA can be eliminated. However, if the sample is treated with the tetrazolium compound, not only the effect of the low-molecular-weigh reducing substance but also the effect of the high-molecular-weigh reducing substance, especially hemoglobin and a hemoglobin degradation product (hereinafter, both of them are altogether referred to as "hemoglobin"), can be eliminated. The applicant of the present invention filed another application (JP 2000-210100 A) on this finding. However, in light of the fact that the accuracy of the determination cannot be improved sufficiently according to the method disclosed in JP 2000-210100 A although the effect of the high-molecular-weight reducing substance, e. g., hemoglobin and the like, is eliminated and that still further improvement in the accuracy of the determination is desired, the inventors of the present invention have made a further intensive study. As a result, the inventors of the present invention have found that, although the effect of the high-molecular-weight reducing substance, e.g., hemoglobin and the like, can be eliminated by treating the sample with the tetrazolium compound, cloudiness is generated in the reaction solution due to the coexistence of the high-molecular-weight reducing substance and the tetrazolium compound. Further, the inventors of the present invention have found that the generation of such cloudiness can be prevented by a surfactant, thereby establishing the present invention. According to the method of the present invention, the effect of the reducing substance is eliminated by the tetrazolium compound, and in addition, the generation of the cloudiness caused by the treatment with the tetrazolium compound is prevented. Thus, interference with determination caused by both the reducing substance and the cloudiness can be prevented, thereby allowing an analyte in a sample to be determined with even higher accuracy. Therefore, the method of the present invention is useful for various tests in clinical medical services etc. as described above, and in particular, by using this method to determine glycated hemoglobin, the reliability of the glycated hemoglobin as an index for the diagnosis etc. of diabetes can be improved.

In the method of the present invention, it is preferable that the sample contains hemoglobin and a hemoglobin degradation product and that an effect of the hemoglobin and the hemoglobin degradation product as reducing substances is eliminated. This allows especially the effect of the hemoglobin as a reducing substance in the sample to be eliminated and also allows the cloudiness due to the coexistence of the hemoglobin and the tetrazolium compound to be eliminated. Therefore, the method of the present invention according to this preferable example is useful in the case where the sample is any one of blood samples as described later.

In the method of the present invention, the tetrazolium compound may be added to the sample first and then, the surfactant may further be added to the resultant mixture in which cloudiness has been generated. However, it is preferable that the tetrazolium compound and the surfactant are added at the same time or the tetrazolium compound is added to the sample after the surfactant has been added to the sample so as to allow the generation of the cloudiness to be prevented.

In the method of the present invention, it is preferable that the determination utilizing the redox reaction is measurement of an absorbance of a color-developing substance produced by the redox reaction.

In general, dual-wavelength measurement is used dominantly for measuring the absorbance. The dual-wavelength measurement is carried out in the following manner, for example. First, the absorbance of a substance to be measured (e.g., a color-developing substance such as a color-developing substrate and the like) is measured at a main wavelength at which the substance exhibits the maximum absorption. Then, at a sub-wavelength that is different from the main wavelength, electrical noises, cloudiness of the sample, changes in the amount of light, etc. are measured to correct the measured value obtained at the main wavelength. Therefore, it is preferable that the sub-wavelength is a wavelength at which a substance other than the substance to be measured (the color-developing substance) contained in the sample exhibits no absorption and that the distance between the sub-wavelength and the main wavelength is at least about 80 nm. Accordingly, the inventor of the present invention carried out a further study and found the following. In the case where Hb is treated with a tetrazolium compound in the absence of a surfactant, the products obtained through the reaction exhibit absorption at the wavelength in the range from about 700 to 900 nm, especially from about 760 to 900 nm. Thus, it is difficult to measure the absorbance of the color-developing substance at a wavelength in this range. In contrast, in the case where Hb is treated with a tetrazolium compound in the presence of a surfactant, no absorption is observed at a wavelength in the above-mentioned range. Therefore, by setting the measurement wavelengths (the main wavelength and the sub-wavelength) for measuring the absorbance of the color-developing substance in the range from about 700 to 900 nm, not only is the cloudiness prevented from being generated, but also the effect of the absorption by Hb can be eliminated, thereby allowing the absorbance of the color-developing substance to be measured accurately.

In the method of the present invention, it is preferable that the measurement of the absorbance is carried out at a wavelength in a range from 650 to 900 nm, more preferably from 650 to 800 nm, and most preferably from 690 to 760 nm. Further, in the case where the wavelength in the above-mentioned range is used as the main wavelength, it is preferable that the sub-wavelength is greater than the main wavelength. For example, the sub-wavelength preferably is in the range from 730 to 900 nm, more preferably from 800 to 900 nm, and most preferably from 800 to 850 nm.

In the method of the present invention, it is preferable that the surfactant is at least one surfactant selected from the group consisting of nonionic surfactants, alkyl sulfate, and high-molecular compounds.

Examples of the nonionic surfactants include polyoxyethylene ethers, sorbitan fatty acid esters, and the like. Among these, polyoxyethylene ethers are preferable.

Polyoxyethylene ethers, which are represented by [$C_LH_M$-O-$(CH_2CH_2O)_NH$], are compounds in which a polyoxyethylene chain and a hydrocarbon chain are linked with each other by an ether linkage. Examples of the hydrocarbon

chain include an alkyl group, an alkyl phenyl group, and the like. Preferably, the polyoxyethylene chain has a weight-average degree of polymerization (N) in the range from 8 to 23 and the hydrocarbon chain has a carbon number (L) in the range from 8 to 18. More preferably, the polyoxyethylene chain has a weight-average degree of polymerization (N) in the range from 8 to 15 and the hydrocarbon chain has a carbon number (L) in the range from 8 to 16. Most preferably, the polyoxyethylene chain has a weight-average degree of polymerization (N) in the range from 8 to 10 and the hydrocarbon chain has a carbon number (L) in the range from 8 to 14. For example, the hydrocarbon chain may be a straight chain or may have a branched chain. Specific examples of the polyoxyethylene ethers include polyoxyethylene-p-t-octylphenyl ether, polyethylene glycol (10) lauryl ether, and polyethylene glycol (9) lauryl ether.

Examples of the sorbitan fatty acid esters include polyoxyethylene sorbitan alkyl ester and the like.

Examples of the alkyl sulfate include sodium lauryl sulfate, laurylbenzenesulfonic acid sodium salt, 2,4-dimethylbenzene sulfonic acid sodium salt, and the like.

Examples of the high-molecular compounds include biopolymer compounds such as water-soluble gelatinum, pullulan, and the like and synthesized high-molecular compounds such as polyethylene glycol, polyvinyl pyrrolidone, and the like. Polyethylene glycol has a weight-average degree of polymerization in the range from 100 to 30,000, preferably in the range from 600 to 6000, for example. Pullulan is a polysaccharide. More specifically, pullulan is a water-soluble $\alpha$-glucan in which maltotriose residues are linked with each other by $\alpha$(1-6) linkage.

In the method of the present invention, it is preferable that the surfactant is added to the sample so that a content of the surfactant per milliliter of the sample is in the range from 0.05 to 5 mmol, more preferably from 0.1 to 3 mmol, and most preferably from 0.2 to 1.5 mmol. Further, it is preferable that the surfactant is added to the sample so that a content of the surfactant per mole of the tetrazolium compound is in the range from 0.2 to 15 mmol, more preferably from 0.5 to 10 mmol, and most preferably from 0.7 to 5 mmol.

The tetrazolium compound to be used in the method of the present invention preferably contains ring substituents at least at two positions on its tetrazole ring, more preferably at three positions on its tetrazole ring, for example.

In the case where the tetrazolium compound contains ring substituents at least at two positions on its tetrazole ring as described above, it is preferable that the ring substituents are at the 2-position and 3-position on the tetrazole ring. Further, in the case where the tetrazolium compound contains ring substituents at three positions on its tetrazole ring, it is preferable that the ring substituents are at the 2-position, 3-position, and 5-position on the tetrazole ring.

Further, it is preferable that at least two ring substituents of the tetrazolium compound have a benzene ring structure. Other than the benzene ring structure, the ring substituents may have a resonance structure with S or O being contained in the ring skeleton, for example. Examples of the ring substituents with such a resonance structure include a thienyl group, thiazoyl group, and the like.

Furthermore, it is preferable that the tetrazolium compound contains ring substituents at least at three positions on its tetrazole ring and at least two of the ring substituents have a benzene ring structure.

Still further, it is preferable that at least one ring substituent contains a functional group, and a larger number of functional groups are more preferable.

As the functional group, an electron-withdrawing functional group preferably is used. For example, a halogen group, ether group, ester group, carboxy group, acyl group, nitroso group, nitro group, hydroxy group, sulfo group, and the like can be used. Other than these, characteristic groups containing oxygen such as a hydroperoxy group, oxy group, epoxy group, epidioxy group, oxo group, and the like; and characteristic groups containing sulfur such as a mercapto group, alkylthio group, methylthiomethyl group, thioxo group, sulfino group, benzenesulfonyl group, phenylsulfonyl group, p-toluenesulfonyl group, p-tolylsulfonyl group, tosyl group, sulfamoyl group, isothiocyanate group, and the like also can be used, for example. Among these electron-withdrawing functional groups, a nitro group, sulfo group, halogen group, carboxy group, hydroxy group, methoxy group, ethoxy group are preferable. Further, in addition to the above-mentioned electron-withdrawing functional groups, unsaturated hydrocarbon groups such as a phenyl group ($C_6H_5$-), styryl group ($C_6H_5CH = CH$-), and the like also can be used, for example. It is to be noted that the functional groups may have been ionized by dissociation.

Still further, it is preferable that the tetrazolium compound contains benzene rings at the 2-position and 3-position on its tetrazole ring and at least one of the benzene rings contains at least one functional group selected from the group consisting of a halogen group, carboxy group, nitro group, hydroxy group, sulfo group, methoxy group, and ethoxy group. It is to be noted here that both the benzene rings may contain the functional group. Further, the functional group may be contained at any positions (ortho-, meta-, para-) on the benzene ring. Furthermore, the number of the functional group is not specifically limited, and in the case where the at least one of the benzene rings contains a plurality of functional groups, the functional groups contained in the benzene ring may be the same or different.

Examples of the tetrazolium compound containing ring substituents having a benzene ring structure at the 2-position, 3-position, and 5-position on its tetrazole ring include:

2-(4-iodophenyl)-3-(4-nitrophenyl)-5'(2,4-disulfophenyl)-2H-tetrazolium salt;
2-(4-iodophenyl)-3-(2,4-dinitrophenyl)-5-(2,4-disulfophenyl)-2H-tetrazolium salt;

2-(2-methoxy-4-nitrophenyl)-3-(4-nitrophenyl)-5-(2,4-disulfophenyl)-2H-tetrazolium salt;
2-(4-iodophenyl)-3-(4-nitrophenyl)-5-phenyl-2H-tetrazolium salt;
3,3'-(1,1'-biphenyl-4,4'-diyl)-bis(2,5-diphenyl)-2H-tetrazolium salt;
3,3'-[3,3'-dimethoxy-(1,1'-biphenyl)-4,4'-diyl]-bis[2-(4-nitrophenyl)-5-phenyl-2 H-tetrazolium salt];
2,3-diphenyl-5-(4-chlorophenyl) tetrazolium salt;
2,5-diphenyl-3-(p-diphenyl) tetrazolium salt;
2,3-diphenyl-5-(p-diphenyl) tetrazolium salt;
2,5-diphenyl-3-(4-styrylphenyl) tetrazolium salt;
2,5-diphenyl-3-(m-tolyl) tetrazolium salt; and
2,5-diphenyl-3-(p-tolyl) tetrazolium salt.

The tetrazolium compound is not limited to those described above. In addition to the above-mentioned tetrazolium compounds, a tetrazolium compound containing ring substituents having a benzene ring structure at two positions and one ring substituent having a structure other than the benzene ring structure at one position on its tetrazole ring also may be used. Examples of such a tetrazolium compound include:

2,3-diphenyl-5-(2-thienyl) tetrazolium salt;
2-benzothiazoyl-3- (4-carboxy-2-methoxyphenyl)-5-[4-(2-sulfoethyl carbamoyl) phenyl]-2H-tetrazolium salt;
2,2'-dibenzothiazoyl-5,5'-bis [4-di(2-sulfoethyl) carbamoylphenyl]-3,3'-(3,3'-dimethoxy-4,4'-biphenylene) ditetrazolium salt; and
3'(4,5-dimethyl-2-thiazoyl)-2,5-diphenyl-2H-tetrazolium salt.

Further, a tetrazolium compound containing ring substituents having a benzene ring structure at two positions and one substituent not having a ring structure at one position on its tetrazole ring also can be used.
Examples of such a tetrazolium compound include:

2,3-diphenyl-5-cyano tetrazolium salt;
2,3-diphenyl-5-carboxy tetrazolium salt;
2,3-diphenyl-5-methyltetrazolium salt; and
2,3-diphenyl-5-ethyl tetrazolium salt.

Among the above-mentioned tetrazolium compounds, the tetrazolium compounds containing three ring substituents are preferable as described above. Among these, the tetrazolium compounds containing three ring substituents having a benzene ring structure and a large number of electron-withdrawing functional groups is more preferable, and
2-(4-iodophenyl)-3-(2,4-dinitrophenyl)-5-(2,4-disulfophenyl)-2H-tetrazolium salt is most preferable.

It is to be noted here that the above-mentioned tetrazolium compounds may be a salt or may have been ionized, for example. Further, the tetrazolium compound to be used in the method of the present invention is not limited to only one type, and two or more types of the tetrazolium compounds may be used in combination.

In the method of the present invention, the amount of the tetrazolium compound to be added to a sample is not specifically limited and may be decided as appropriate, for example, depending on the type of the sample, the amount of hemoglobin and other reducing substances contained in the sample, etc. More specifically, the tetrazolium compound preferably is added to the sample so that a content of the tetrazolium compound per microliter of the sample is in the range from 0.001 to 100 μmol, more preferably from 0.005 to 10 μmol, and most preferably from 0.01 to 1 μmol.

In the method of the present invention, in the case where the sample is whole blood, the tetrazolium compound preferably is added to the whole blood so that a content of the tetrazolium compound per microliter of the whole blood is in the range from 0.001 to 10 μmol, more preferably from 0.005 to 5 μmol, and most preferably from 0.01 to 2 μmol. In general, it is estimated that whole blood contains 50 vol% of blood cells. More specifically, in the case where the tetrazolium compound is 2-(4-iodophenyl)-3-(2,4-dinitrophenyl)-5-(2,4-disulfophenyl)-2H-tetrazolium salt, the tetrazolium compound preferably is added to the whole blood so that a content of the tetrazolium compound per microliter of the whole blood is in the range from 0.02 to 10 μmol, more preferably from 0.05 to 3 μmol, and most preferably from 1 to 2 μmol.

Further, depending on the amount of the tetrazolium compound added to the sample, the amount of the surfactant to be added can be adjusted so as to satisfy the above-mentioned molar ratio, for example.

In the present invention, the above-mentioned measurement of an absorbance of a color-developing substance produced by the redox reaction preferably is the measurement of an absorbance of a color-developing substrate (i.e., color-developing substance) that has developed color as a result of a redox reaction caused by an oxidoreductase between the reducing or oxidizing substance and the color-developing substrate.

More specifically, it is preferable that the oxidizing substance derived from the analyte is hydrogen peroxide, a color-developing substrate that develops color by oxidation is used as the color-developing substrate, and a redox reaction between the hydrogen peroxide and the color-developing substrate is caused by an oxidoreductase.

The oxidoreductase is not specifically limited, and POD is preferably is used as the oxidoreductase, for example. Further, as the color-developing substrate that develops color by oxidation, N-(carboxymethylaminocarbonyl)-4,4'-bis(dimethylamino)diphenylamine sodium preferably is used because it can be detected with high sensitivity.

In the method of the present invention, the sample to be used is not specifically limited. For example, in addition to blood samples such as whole blood, plasma, serum, blood cells, and the like, biological samples such as urine, spinal fluid, saliva and the like; beverage such as juice and the like; food such as soy sauce, sauce, and the like also can be used as the sample. Further, when determining a blood cell component, the sample may be whole blood subjected to a treatment for causing hemolysis (hereinafter, referred to as "hemolysis treatment") or red blood cells separated from whole blood and then subjected to a hemolysis treatment.

In the method of the present invention, the analyte is not specifically limited as long as it is determined utilizing the redox reaction. For example, whole blood components, red cell components, plasma components, serum components, and the like may be the analyte. Among these, red cell components are preferable. More specifically, the analyte may be, for example, glycated proteins such as glycated hemoglobin, glycated albumin, and the like, glycated peptides, glycated amino acids, glucose, uric acid, cholesterol, creatinine, sarcosine, glycerol, and the like. Among these, glycated proteins are more preferable, and glycated hemoglobin is most preferable.

In the method of the present invention, in the case where the analyte is a glycated protein, it is preferable that a glycation site thereof is oxidatively degraded by FAOD to generate hydrogen peroxide. Also, in the case where the analyte is a glycated peptide or glycated amino acid, it is preferable that FAOD similarly acts on a glycation site thereof. It is preferable that the glycated protein and the glycated peptide are treated with a protease prior to the FAOD treatment, if necessary.

As the FAOD, FAOD catalyzing a reaction represented by Formula (1) below preferably is used.

$$R^1\text{-CO-CH}_2\text{-NH-}R^2 + H_2O + O_2$$
$$\rightarrow R^1\text{-CO-CHO} + NH_2\text{-}R^2 + H_2O_2 \qquad (1)$$

In Formula (1), $R^1$ denotes a hydroxyl group or a residue derided from a sugar that is not yet subjected to the glycation reaction (i.e., sugar moiety). The sugar moiety ($R^1$) is an aldose residue when the unreacted sugar is aldose, and is a ketose residue when the unreacted sugar is ketose. When the unreacted sugar is glucose, for example, the sugar in the glycated product takes on the fructose structure after the glycation reaction due to Amadori rearrangement. In this case, the sugar moiety ($R^1$) is a glucose residue (aldose residue). This sugar moiety ($R^1$) can be represented, for example, by

$$\text{-[CH(OH)]}_n\text{-CH}_2\text{OH}$$

where n denotes an integer of 0 to 6.

In Formula (1), $R^2$ is not specifically limited. However, in the case where the substrate is a glycated amino acid, a glycated peptide, or a glycated protein, for example, $R^2$ varies depending on which of an $\alpha$-amino group and an amino group other than the $\alpha$-amino group is glycated.

In Formula (1), in the case where an $\alpha$-amino group is glycated, $R^2$ is an amino acid residue or a peptide residue represented by Formula (2) below.

$$\text{-CHR}^3\text{-CO-R}^4 \qquad (2)$$

In Formula (2), $R^3$ denotes an amino-acid side chain group. $R^4$ denotes a hydroxyl group, an amino acid residue, or a peptide residue, and can be represented, for example, by Formula (3) below. In Formula (3), n denotes an integer of 0 or more and $R^3$ denotes an amino-acid side chain group as described above.

$$\text{-(NH-CHR}^3\text{-CO)}_n\text{-OH} \qquad (3)$$

In Formula (1), in the case where an amino group other than the α-amino group is glycated (i.e., an amino-acid side chain group is glycated), $R^2$ is represented by Formula (4) below.

$$-R^5-CH(NH-R^6)-CO-R^7 \quad (4)$$

In Formula (4), $R^5$ denotes a portion other than the glycated amino group in the amino-acid side chain group. For example, in the case where the glycated amino acid is lysine, $R^5$ is as follows.

$$-CH_2-CH_2-CH_2-CH_2-$$

On the other hand, in the case where the glycated amino acid is arginine, for example, $R^5$ is as follows.

$$-CH_2-CH_2-CH_2-NH-CH(NH_2)-$$

In Formula (4), $R^6$ denotes hydrogen, an amino acid residue, or a peptide residue, and can be represented, for example, by Formula (5) below. In Formula (5), n denotes an integer of 0 or more and $R^3$ denotes an amino-acid side chain group as described above.

$$-(CO-CHR^3-NH)_n-H \quad (5)$$

In Formula (4), $R^7$ denotes a hydroxyl group, an amino acid residue, or a peptide residue, and can be represented, for example, by Formula (6) below. In Formula (6), n denotes an integer of 0 or more and $R^3$ denotes an amino-acid side chain group as described above.

$$-(NH-CHR^3-CO)_n-OH \quad (6)$$

<u>Brief Description of Drawings</u>

FIG. 1 is a graph showing the change in absorbance with time in the case where a hemolysate sample is reacted with WST-3 in the presence of Triton X-100 in a method of determining an analyte according to one example of the present invention.
FIG. 2 is a graph showing the change in absorbance with time in the case where a hemolysate sample is reacted with WST-3 in the presence of Tween 20 in the method of determining an analyte according to the same example.
FIG. 3 is a graph showing the change in absorbance with time in the case where a hemolysate sample is reacted with WST-3 in the presence of polyethylene glycol lauryl ether in the method of determining an analyte according to the same example.
FIG. 4 is a graph showing the change in absorbance with time in the case where a hemolysate sample is reacted with WST-3 in the absence of a surfactant in a method of determining an analyte according to a comparative example of the present invention.

<u>Best Mode for Carrying Out the Invention</u>

Hereinafter, a method of determining an analyte in a sample utilizing a redox reaction according to the present invention will be described by taking as an example the case where a glycated protein in blood cells is determined.
First, a hemolysate sample is prepared by causing hemolysis of whole blood or of a blood cell fraction separated from the whole blood according to a usual method such as centrifugation and the like. The method of causing hemolysis is not specifically limited, and can be, for example, a method using a surfactant, a method using ultrasonic waves, and a method utilizing the difference in osmotic pressure. Among these, the method using a surfactant is preferable.
The surfactant to be used for causing hemolysis is not specifically limited. However, the same surfactant as

used in a pretreatment with a tetrazolium compound as described later preferably is used on account of the ease of operation. Generally, the above-mentioned hemolysis treatment can be carried out under the following conditions: in the case where the solution to be treated contains 1 to 10 vol% of blood cells, the surfactant is added to the solution so as to give a concentration of 0.01 to 5 wt% and the resultant mixture is stirred at room temperature for about a few seconds (about 5 seconds) to 10 minutes.

Subsequently, the hemolysate sample is pretreated by adding the tetrazolium compound having a tetrazole ring structure as described above in the presence of the surfactant.

As the surfactant, the above-mentioned various surfactants can be used. More specifically, polyoxyethylene-p-t-octylphenyl ether such as commercially available Triton-type surfactants and the like; polyoxyethylene sorbitan alkyl ester such as commercially available Tween-type surfactants and the like; and polyoxyethylene alkyl ether such as commercially available Brij-type surfactants and the like can be used, for example. Other than these, polyoxyethylene (10) lauryl ether; polyoxyethylene (9) lauryl ether such as Nikkol BL-9 EX (trade name, available from Wako Pure Chemical Industries, Ltd.: the weight-average degree of polymerization N of polyoxyethylene is 9) and the like; and polyoxyethylene octylphenyl ether such as Tergitol NPX (trade name, available from Nacalai Tesque, Inc.: the weight-average degree of polymerization N of polyoxyethylene is about 10.5), Tergitol NP-40 (trade name, available from Nacalai Tesque, Inc.: the weight-average degree of polymerization N of polyoxyethylene is 20), and the like also can be used, for example.

The surfactant is added to the sample at the same ratio as described above. More specifically, in the case where the solution to be pretreated contains 1 to 10 vol% of blood cells, the surfactant is added to the solution so as to give a concentration in the range from 2 to 150 mmol/L, preferably from 5 to 100 mmol/L, and most preferably from 10 to 50 mmol/L. Further, in the case where the preparation of the hemolysate sample (i.e., the hemolysis treatment step) is carried out using the same surfactant as used in this pretreatment, the surfactant previously may be added to the sample during this hemolysis treatment so as to give a sufficient surfactant concentration for this pretreatment. Also, to the sample that has not yet been subjected to the hemolysis treatment, the surfactant may be added together with a tetrazolium compound, thereby carrying out the hemolysis treatment and the pretreatment at the same time.

As the tetrazolium compound, the tetrazolium compounds as described above can be used. The tetrazolium compound is added to the sample at the following ratio, for example: in the case where the solution to be pretreated contains 1 to 10 vol% of blood cells, the tetrazolium compound preferably is added to the solution so as to give a concentration in the range from 0.02 to 2000 mmol/L, more preferably from 0.1 to 1000 mmol/L, and most preferably from 0.4 to 200 mmol/L. More specifically, in the case where the tetrazolium compound is 2-(4-iodophenyl)-3-(2,4-dinitrophenyl)-5-(2,4-disulfophenyl)-2H-tetrazolium salt, the tetrazolium compound preferably is added to the solution so as to give a concentration in the range from 0.02 to 80 mmol/L, more preferably from 0.1 to 20 mmol/L, and most preferably from 0.2 to 15 mmol/L.

The tetrazolium compound may be used as it is. However, on account of the ease of operation, treatment efficiency, etc., it is preferable to use a tetrazolium compound solution obtained by dissolving the tetrazolium compound in a solvent. The concentration of the tetrazolium compound in the solution can be decided as appropriate depending on the type of the tetrazolium compound (e.g., the molecular weight thereof) and the like. For example, the concentration of the tetrazolium compound in the solution is in the range from 0.01 to 120 mmol/L, preferably from 0.1 to 50 mmol/L, and more preferably from 0.2 to 20 mmol/L. As the solvent, distilled water, a physiological salt solution, buffers, and the like can be used, for example. Examples of the buffers include those described in the following.

Generally, this pretreatment is carried out in a buffer. Examples of the buffer includes amine buffers, phosphate buffers, borate buffers, and Good's buffers such as CHES buffer, CAPS buffer, CAPSO buffer, and the like. Among these, amine buffers and CHES buffer are preferable. Examples of buffer agents contained in the amine buffers include glycine, ethylamine, diethylamine, methylamine, dimethylamine, trimethylamine, tris(hydroxymethyl) aminomethane, triethanolamine, glycineamide, and the like. Among these, glycine, glycineamide, and triethanolamine are preferable.

The pH of the above buffers preferably is in the range from 7 to 12, more preferably from 8 to 11, and most preferably from 8 to 10.

The conditions of this pretreatment are not specifically limited. However, the pretreatment generally is carried out at a temperature in the range from 10°C to 37°C and the treatment time in the range from 10 seconds to 60 minutes.

Next, the pretreated hemolysate sample is treated with a protease. This protease treatment is carried out so that FAOD to be used in a subsequent treatment can act on a glycation site of the analyte easily.

Examples of the protease include serine proteases, thiol proteases, metalloproteinases, and the like. More specifically, a metalloprotease, trypsin, proteinase K, chymotrypsin, papain, bromelain, subtilisin, elastase, aminopeptidase, pepsine, and the like can be used.

Further, in the case where the glycated protein is glycated hemoglobin, a protease that degrades the glycated Hb selectively, e.g., a bromelain, papain, trypsin derived from porcine pancreas, metalloproteinase, and protease derived from *Bacillus subtilis*, preferably are used. Among these, a metalloproteinase, bromelain, and papain are more preferable, and a metalloproteinase is most preferable. By using the protease that degrades the glycated Hb selectively,

the accuracy of determination can be further improved because a glycated Hb degradation product can be prepared selectively and the glycated proteins other than the glycated Hb are hardly degraded. Examples of the protease derived from *Bacillus subtilis* include Protease N (trade name, available from Fluka Chemie AG, for example), Protease N "AMANO" (trade name, available from Amano Enzyme Inc.), and the like. Examples of the metalloproteinase include the metalloproteinase (EC 3. 4. 24. 4) derived from the genus *Bacillus* (e.g., available from Toyobo Co., Ltd. under the trade name Toyoteam) and the like.

The conditions of the protease treatment are decided as appropriate, for example, depending on the type of the protease to be used, the type and the concentration of the glycated protein as an analyte, and the like.

More specifically, in the case where the pretreated hemolysate sample is treated using Protease K as the protease, for example, the treatment generally is carried out under the following conditions: the protease concentration in the reaction solution in the range from 10 to 30,000 mg/L (1 KU/L to 250 MU/L), the blood cell concentration in the reaction solution in the range from 0.05 to 15 vol%, the reaction temperature in the range from 15°C to 37°C, the reaction time in the range from 1 minute to 24 hours, and the pH in the range from 6 to 12. Generally, this protease treatment is carried out in a buffer. As the buffer, the same buffers as used in the above-mentioned pretreatment also can be used.

Further, in the case where the pretreated hemolysate sample is treated using a metalloprotease as the protease, for example, the treatment generally is carried out under the following conditions: the protease concentration in the reaction solution in the range from 0.02 to 6 g/L (40 KU/L to 40 MU/L), the blood cell concentration in the reaction solution in the range from 0.05 to 15 vol%, the reaction temperature in the range from 15°C to 37°C, the reaction time in the range from 1 minute to 24 hours, and the pH in the range from 6 to 12.

Next, the degradation product obtained through the above-mentioned protease treatment is treated with the FAOD. This FAOD treatment catalyzes the reaction represented by Formula (1) above.

Similarly to the above-mentioned protease treatment, this FAOD treatment preferably is carried out in a buffer. The conditions of the FAOD treatment are decided as appropriate depending on the type of the FAOD to be used, the type and the concentration of the glycated protein as an analyte, and the like.

More specifically, the FAOD treatment is carried out, for example, under the following conditions: the FAOD concentration in the reaction solution in the range from 50 to 50,000 U/L, the blood cell concentration in the reaction solution in the range from 0.01 to 1 vol%, the reaction temperature in the range from 15°C to 37°C, the reaction time in the range from 1 to 60 minutes, and the pH in the range from 6 to 9. The type of the buffer is not specifically limited, and the same buffers as used in the protease treatment also can be used in the FAOD treatment.

Next, using an oxidoreductase and the color-developing substrate that develops color by oxidation, the amount of the hydrogen peroxide generated by the FAOD treatment is determined utilizing a redox reaction.

As the oxidoreductase, POD and the like can be used, for example.

As the color-developing substrate, N-(carboxymethylaminocarbonyl)-4,4'-bis(dimethylamino)diphenylamine sodium, orthophenylenediamine (OPD), a substrate obtained by combining a Trinder's reagent and 4-aminoantipyrine, and the like can be used, for example. Examples of the Trinder's reagent include phenols, phenol derivatives, aniline derivatives, naphthols, naphthol derivatives, naphthylamine, naphthylamine derivatives, and the like. Further, in place of the above-mentioned aminoantipyrine, it is possible to use aminoantipyrine derivatives, vanillin diamine sulfonic acid, methyl benzothiazolinone hydrazone (MBTH), sulfonated methyl benzothiazolinone hydrazone (SMBTH), and the like. Among these color-developing substrates, N-(carboxymethylaminocarbonyl)-4,4'-bis (dimethylamino)diphenylamine sodium is most preferable as described above.

The above-mentioned redox reaction generally is induced in a buffer under the conditions decided as appropriate depending on the concentration of the hydrogen peroxide generated and the like. Generally, the redox reaction is induced under the following conditions: the oxidoreductase concentration in the reaction solution in the range from 10 to 100,000 IU/L, the concentration of the color-developing substrate in the range from 0.005 to 30 mmol/l, the reaction temperature in the range from 15°C to 37°C, the reaction time in the range from 6 seconds to 30 minutes, and the pH in the range from 5 to 9. Further, the buffer is not specifically limited, and the same buffers as used in the protease treatment, the FAOD treatment, etc. also can be used.

In the case where the color-developing substrate, for example, is used in the redox reaction, the amount of the hydrogen peroxide can be determined by measuring the degree of color development (i.e., the absorbance) of the reaction solution with a spectrophotometer. The amount of the glycated protein in the sample can be determined using the concentration of the hydrogen peroxide thus determined and a calibration curve etc.

It is to be noted here that the amount of the hydrogen peroxide can be determined not only by the above-mentioned enzymic method using the POD etc. but also by an electrical method, for example.

Further, in the method of determining an analyte in a sample utilizing a redox reaction according to the present invention, the order in which the pretreatment with the tetrazolium compound is carried out is not specifically limited as long as it is carried out before the redox reaction occurs substantially. However, since hydrogen peroxide is generated after the FAOD treatment, the pretreatment with the tetrazolium compound preferably is carried out prior to the FAOD

treatment. Further, the respective treatment steps may be performed individually as described above, or some of the treatment steps may be performed simultaneously in the following combinations, for example.

1: hemolysis treatment + pretreatment
2: hemolysis treatment + pretreatment + protease treatment
3 : protease treatment + FAOD treatment
4: FAOD treatment + color-developing reaction
5: protease treatment + FAOD treatment + color-developing reaction

Also, the order in which the tetrazolium compound and the surfactant are added and the order in which the FAOD, the oxidoreductase, and the color-developing substrate are added are not specifically limited.

According to the method of the present invention, not only the effect of a low-molecular-weight reducing substance such as GSH, AsA, dithiothreitol, cysteine, N-acetyl-cysteine, and the like but also the effect of especially hemoglobin and glycated hemoglobin as a reducing substance can be eliminated sufficiently by bringing the tetrazolium compound into contact with the sample. In addition, the cloudiness due to the coexistence of the tetrazolium compound and hemoglobin can be prevented. Therefore, it becomes possible to determine an analyte in the sample accurately without causing any effect on the above-mentioned measurement of the absorbance, for example.

Further, in the pretreatment with the tetrazolium compound, the tetrazolium compound may be used in combination with an oxidizing agent other than the tetrazolium compound. Examples of the oxidizing agent include halogen oxides such as sodium iodoacetate, iodic acid, periodic acid, and the like, EDTA-Fe, ascorbate oxidase, bilirubin oxidase, and the like. Such an oxidizing agent is added to a sample so that a content of the oxidizing agent per microliter of the sample is in the range from 0.001 to 0.1 mg.

In the method of determining an analyte in a sample utilizing a redox reaction according to the present invention, the analyte is not specifically limited as long as it can be determined utilizing a redox reaction. Examples of the analyte include glycated peptides, glycated amino acids, glucose, uric acid, cholesterol, creatinine, sarcosine, glycerol, and the like, in addition to the above-mentioned glycated proteins, as described above.

In the case where the amount of the above-mentioned respective analytes is determined by causing hydrogen peroxide to be generated, it is preferable that the hydrogen peroxide is generated by having glucose oxidase act on glucose; cholesterol oxidase act on cholesterol; uricase act on uric acid; sarcosine oxidase act on creatinine; and glycerol oxidase act on glycerol. The amount of the hydrogen peroxide generated can be determined in the same manner as described above. Further, the glycated peptides and the glycated amino acids can be determined in the same manner as that for determining the glycated proteins.

Furthermore, in the case where the amount of the analyte is determined by: causing a reducing substance derived from the analyte to be produced after treating hemoglobin and a hemoglobin degradation product in the sample with the tetrazolium compound; determining the amount of the reducing substance derived from the analyte utilizing a redox reaction; and then determining the amount of the analyte from the amount of the reducing substance thus determined, the determination can be carried out, for example, in the following manner.

For example, in the case where the analyte is glucose, reducing substances such as NADH, NADPH, and the like are produced by using glucose dehydrogenase in the presence of $NAD^+$, $NADP^+$, and the like, for example. Thereafter, NADH and NADPH as the reducing substances derived from the analyte is determined by causing a redox reaction using diaphorase and a substrate that develops color by reduction, for example. Then, the amount of the analyte in the sample can be determined using the concentration of the reducing substances derived from the analyte and a calibration curve etc., as described above. In the case where the analyte is cholesterol, cholesterol dehydrogenase can be used. In the case where the analyte is sarcosine, sarcosine dehydrogenase can be used.

The substrate that develops color by reduction is not specifically limited, and can be, for example, a color-developing tetrazolium compound added to the sample to eliminate the effect of hemoglobin and a hemoglobin product in the sample. Further, in accordance with the measurement wavelength to be used, a color-developing tetrazolium compound that is different from the tetrazolium compound used in the pretreatment of the sample may be used. In addition to the color-developing tetrazolium compound as described above, 2,6-dichlorophenolindophenol and the like also can be used. Furthermore, in order to obtain a determined value with a higher reliability, it is preferable that the absorbance is determined prior to determining a reducing substance derived from the analyte, for example.

## EXAMPLES

Hereinafter, examples of the present invention will be described along with comparative examples.

Example 1

In Example 1, blood cell samples were pretreated with a tetrazolium compound in the presence of various surfactants to examine the presence or absence of cloudiness. The samples, reagents, and method used in the present example will be described in the following.

(Preparation of Samples)

Whole blood collected from a healthy subject was centrifuged (1500 G (3000 rpm), 3 min) and blood cells were collected. The blood cells were diluted with 31 times its volume of purified water to cause hemolysis. The resultant solution was used as the blood cell samples.

(Surfactant Solutions)

Surfactants shown in Table 1 below were dissolved in purified water, respectively, to prepare 2.4 wt% solutions of the respective surfactants.

Among the surfactants shown in Table 1, Triton X-100 (trade name), Brij 35 (trade name), Nikkol BL-9 EX (trade name), and 2,4-dimethylbenzene sulfonic acid sodium salt are available from Wako Pure Chemical Industries, Ltd.; Triton X-114 (trade name), Triton N-101 (trade name), Tween 20 (trade name), Tergitol NPX (trade name), Tergitol NP-40 (trade name), polyethylene glycol lauryl ether, laurylbenzenesulfonic acid sodium salt, PEG 1000 (trade name), PEG 6000 (trade name) are available from Nacalai Tesque, Inc.; Brij 58 (trade name), Brij 98 (trade name), and Arlasolve 200 are available from Sigma Chemical Co.; and Pullulan PI-20 (trade name) is available from Kabushiki Kaisha Hayashibara Seibutsu Kagaku Kenkyujo.

(Buffer)

| | |
|---|---|
| CHES buffer (pH 9.0) | 0.2 mol/L |

(WST-3 solution: hereinafter the same)

2-(4-iodophenyl)-3- (2,4-dinitrophenyl)-5-(2,4-disulfophenyl)-2H-tetrazolium monosodium salt (available from Dojindo Laboratories under the trade name WST-3) represented by Formula (7) below was dissolved in purified water so as to give a concentration of 1.66 mmol/L to prepare a WST-3 solution.

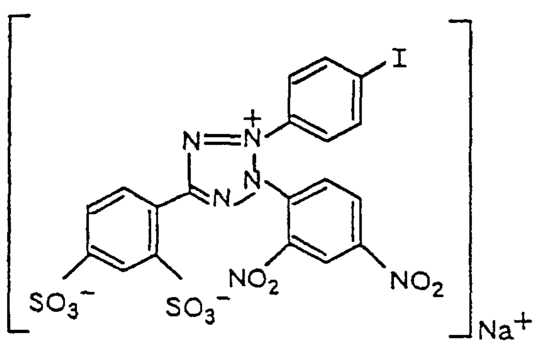

WST-3 (7)

(Method for Checking Cloudiness)

150 µL of each of the surfactant solutions, 300 µL of the buffer, and 900 µL of the WST-3 solution were mixed with 345 µL of the respective samples, and the mixtures were incubated at 37°C for 5 minutes. The cloudiness in the respective mixtures was checked visually and evaluated according to the following evaluation criteria. Further, as a comparative example, the cloudiness of the mixture prepared in the same manner as described above except that

purified water was added in place of the surfactant was evaluated in the same manner. The results are shown in Table 1 below.

(Evaluation Criteria for Cloudiness)

○: no cloudiness was generated
×: cloudiness was generated

(Table 1)

| Surfactant | Cloudiness Evaluation |
|---|---|
| (Comparative Example 1) | |
| No surfactant | × |
| (Example 1) | |
| Triton X-100 | ○ |
| Triton X-114 | ○ |
| Triton N-101 | ○ |
| Tween 20 | ○ |
| Brij 35 | ○ |
| Brij 58 | ○ |
| Brij 98 | ○ |
| Tergitol NPX | ○ |
| Tergitol NP-40 | ○ |
| Arlasolve 200 | ○ |
| polyethylene glycol lauryl ether | ○ |
| deoxycholic acid | ○ |
| Nikkol BL-9 EX | ○ |
| sodium lauryl sulfate | ○ |
| laurylbenzenesulfonic acid sodium salt | ○ |
| 2,4-dimethylbenzene sulfonic acid sodium salt | ○ |
| Pullulan PI-20 | ○ |
| PEG 1000 | ○ |
| PEG 6000 | ○ |

As can be seen from Table 1, the cloudiness was prevented from being generated by treating the samples with the WST-3 in the presence of the surfactants.

Example 2 and Comparative Example 2

In Example 2, samples containing different amounts of surfactants were treated with a tetrazolium compound. The samples, reagents, method, etc. used in the present example will be described in the following.

(Preparation of Samples)

Blood cells (hemoglobin concentration: about 300 g/L) were collected in the same manner as in Example 1 and diluted with 22 times its volume of purified water to cause hemolysis. The resultant solution (hemoglobin concentration: about 13.6 g/L) was used as samples.

(Surfactant Solutions)

The same surfactants as used in Example 1 were dissolved in 0.2 mol/L CHES buffer (pH 9.0), respectively, so as to give predetermined concentrations (1.0 wt% and 2.4 wt%).

The above-mentioned respective samples were diluted 2-fold with purified water. 15 μL of each of the surfactant solutions and 45 μL of the WST-3 solution were mixed with 25 μL of the respective diluted solutions, and the mixtures were incubated at 37°C for 3 minutes. The final concentrations of the surfactants in the mixtures were 0.176 wt% and 0.424 wt%. After the incubation, the absorbance of the mixtures was measured at the wavelength 884 nm and the cloudiness in the mixtures was evaluated in the same manner as in Example 1. Further, as Comparative Example 2, the measurement of the absorbance and the evaluation of the cloudiness were carried out in the same manner as described above under the condition that no surfactant added to the samples. The results are shown in Table 2 below.

(Table 2)

| | Final Concentrations | | |
|---|---|---|---|
| Surfactant | 0.176 wt% (Abs.) | 0.424 wt% (Abs.) | Cloudiness Evaluation |
| (Comparative Example 2) | | | |
| No surfactant | 0.075 | | × |
| (Example 2) | | | |
| Triton X-100 | 0.007 | 0.007 | ○ |
| Triton N-101 | 0.018 | 0.013 | ○ |
| Tween 20 | 0.035 | 0.032 | ○ |
| Brij 35 | 0.017 | 0.012 | ○ |
| Brij 58 | 0.009 | 0.011 | ○ |
| Brij 98 | 0.009 | 0.011 | ○ |
| Tergitol NPX | 0.008 | 0.007 | ○ |
| Tergitol NP-40 | 0.007 | 0.007 | ○ |
| Arlasolve 200 | 0.008 | 0.008 | ○ |
| polyethylene glycol lauryl ether | 0.007 | 0.007 | ○ |
| Nikkol BL-9 EX | 0.015 | 0.011 | ○ |
| sodium lauryl sulfate | 0.011 | 0.017 | ○ |
| laurylbenzenesulfonic acid sodium salt | 0.011 | 0.017 | ○ |
| 2,4-dimethylbenzene salt sulfonic acid sodium salt | 0.012 | 0.012 | ○ |
| Pullulan PI-20 | 0.007 | 0.008 | ○ |
| PEG 1000 | 0.008 | 0.009 | ○ |
| PEG 6000 | 0.007 | 0.008 | ○ |

Further, with regard to the examples using Triton X-100, Tween 20, and polyethylene glycol lauryl ether, respectively, the time charts of the absorbances measured at the wavelengths of 884 nm, 845 nm, and 805 nm are shown in FIGs. 1 to 3, respectively. FIG. 1 is the time chart in the case where Triton X-100 was used; FIG. 2 is the time chart in the case where Tween 20 was used; and FIG. 3 is the time chart in the case where polyethylene glycol lauryl ether was used. As a comparative example, FIG. 4 shows the time chart in the case where no surfactant was added.

As can be seen from Table 2, the cloudiness was prevented from being generated by treating the samples with the WST-3 in the presence of the surfactants. In addition, the absorbances of these samples were kept low as compared with those of the sample to which no surfactant was added. Moreover, while the time chart in FIG. 4 according to the comparative example shows a remarkable increase in the absorbances, the time charts in FIGs. 1 to 3 according to the example in which the surfactants were added show a sufficient decrease in the absorbances, which means the effect of the cloudiness was eliminated.

<u>Example 3 and Comparative Example 3</u>

In Example 3, hemolysate samples to which a glycated amino acid has been added were treated with the WST-3 in the presence of surfactants, and thereafter, the glycated amino acids were determined.

(Redox Reagent)

| | |
|---|---|
| POD (Toyobo Co., Ltd.) | 132 KU/L |
| FAOD (Asahi Chemical Industry Co., Ltd.) | 44 KU/L |
| DA-64 (trade name, Wako Pure Chemical Industries, Ltd.) | 0.088 mmol/L |
| Potassium phosphate buffer (pH 8.0) | 0.2 mol/L |

(Preparation of Glycated Valine)

Glycated valine (Hereinafter, referred to as "FV") was prepared according to a method conventionally known in the art. The FV was dissolved in purified water to prepare a FV solution.

(Surfactant Solutions)

Triton X-100 (trade name), Nikkol BL-9 EX (trade name), and Tween 20 (trade name) were dissolved in 0.2 mol/L CHES buffer (pH 9.0), respectively, so as to give predetermined concentrations (1.0 wt% and 2.4 wt%).

(Preparation of Samples)

Blood cells were collected in the same manner as in Example 1 and diluted with 22 times its volume of purified water to cause hemolysis. The resultant solution was used to prepare samples. As the samples, the following four types of samples (samples a to d) with different Hb concentrations and FV concentrations were prepared.

| | FV concentration (μmol/L) | Hb concentration (g/L) |
|---|---|---|
| Sample a | 9 | 6.8 |
| Sample b | 9 | 13.6 |
| Sample c | 36 | 6.8 |
| Sample d | 36 | 13.6 |

(Determining Method)

To 12.5 μL of the respective samples, 12.5 μL of purified water was added, and then, 15 μL of each of the surfactants was further added. Thereafter, 45 μL of the WST-3 solution was added, and the resultant mixtures were incubated at 37°C for 3 minutes. Then, 25 μL of the redox reagent was added to the mixtures, and the mixtures were allowed to react for 1 minute. After the reaction, the absorbance was measured (at the main wavelength of 751 nm and the sub-wavelength 884 nm). The final concentrations of the surfactants in these reaction solutions were 0.114 wt% and 0.273 wt%. Further, as Comparative Example 3, the absorbance was measured in the same manner except that the CHES buffer was used in place of the surfactants. The results are shown in Table 3 below.

It is to be noted here that the unit "%" used for expressing the concentrations of the surfactants in Table 3 denotes the percent by weight. Further, in Table 3, the values (%) within parentheses denote the percentages (%) of the values (b/a, d/c) obtained by dividing the absorbances of the samples b and d (Hb concentration: 13.6 g/L) by the absorbances of the samples a and c (Hb concentration: 6.8 g/L), respectively. The closer the value to 100%, the more excellent is the accuracy of determination. That is, since the amounts of FV in the samples are equivalent, the absorbances (i.e. the amount of the color developed) of the samples depending on the amount of FV are substantially the same even when the amount of Hb is doubled. Therefore, if the value is close to 100 %, that means the FV is determined accurately while preventing the generation of cloudiness sufficiently.

(Table 3)

| | | | Example 3 | | | | Comparative Example 3 |
|---|---|---|---|---|---|---|---|
| Sample | FV | Hb | TritonX-100 | | Nikkol | BL-9 EX | |
| | μmol/L | μmol/L | 0.114% | 0.273% | 0.114% | 0.273% | |
| Wavelength 751 nm | | | | | | | |
| a | 9 | 6.8 | 0.066 | 0.063 | 0.062 | 0.058 | 0.069 |
| b | 9 | 13.6 | 0.057 | 0.058 | 0.055 | 0.051 | 0.074 |
| c | 36 | 6.8 | 0.202 | 0.184 | 0.201 | 0.201 | 0.192 |
| d | 36 | 13.6 | 0.189 | 0.182 | 0.185 | 0.185 | 0.179 |
| Wavelength 884 nm | | | | | | | |
| a | 9 | 6.8 | 0.004 | 0.004 | 0.004 | 0.004 | 0.019 |
| b | 9 | 13.6 | 0.008 | 0.006 | 0.007 | 0.007 | 0.040 |
| c | 36 | 6.8 | 0.006 | 0.006 | 0.006 | 0.006 | 0.020 |
| d | 36 | 13.6 | 0.009 | 0.008 | 0.008 | 0.008 | 0.044 |
| Wavelength 751nm-884 nm | | | | | | | |
| a | 9 | 6.8 | 0.062 | 0.058 | 0.058 | 0.054 | 0.050 |
| b | 9 | 13.6 | 0.049 | 0.052 | 0.048 | 0.045 | 0.034 |
| | | | (79%) | (89%) | (82%) | (83%) | (68%) |
| c | 36 | 6.8 | 0.197 | 0.179 | 0.195 | 0.195 | 0.172 |
| d | 36 | 13.6 | 0.180 | 0.174 | 0.176 | 0.177 | 0.135 |
| | | | (92%) | (97%) | (90%) | (91%) | (78%) |

As can be seen from Table 3, in Example 3 where the samples were treated with the WST-3 in the presence of the surfactants, the cloudiness was prevented from being generated, and in addition, the absorption by Hb was reduced especially at the wavelength of 884 nm. Therefore, as compared with Comparative Example 3 where the samples were treated with the WST-3 in the absence of the surfactants, the values (%) within parentheses are greater, which means the higher accuracy of determination was achieved.

Industrial Applicability

As specifically described above, the method of present invention enables a highly reliable determination of an analyte in a sample by adding the tetrazolium compound to the sample in the presence of a surfactant because this allows the effect of a reducing substance in the sample to be eliminated and also allows the cloudiness due to the coexistence of the tetrazolium compound and the reducing substance such as hemoglobin to be prevented from being generated. Therefore, the method of the present invention can be applied to various tests in clinical medical services, for example, and is particularly useful for determining a glycated protein, such as glycated hemoglobin in red blood cells, which is important for the diagnosis of diabetes.

## Claims

**1.** A method of determining an analyte in a sample utilizing a redox reaction comprising:

adding a tetrazolium compound to a sample in the presence of a surfactant prior to a redox reaction, thereby eliminating an effect of a reducing substance in the sample;
determining an amount of a reducing or oxidizing substance derived from an analyte utilizing the redox reaction; and
determining an amount of the analyte based on the amount of the reducing or oxidizing substance thus determined.

**2.** The method according to claim 1,

wherein the sample contains hemoglobin and a hemoglobin degradation product, and an effect of the hemoglobin and the hemoglobin degradation product as reducing substances is eliminated.

**3.** The method according to claim 1,
wherein the determination utilizing the redox reaction is measurement of an absorbance of a color-developing substance produced by the redox reaction.

**4.** The method according to claim 3,
wherein the color-developing substance is a color-developing substrate that has developed color as a result of a redox reaction caused by an oxidoreductase between the reducing or oxidizing substance and the color-developing substrate.

**5.** The method according to claim 3,
wherein the measurement of the absorbance is carried out at a wavelength in a range from 650 to 900 nm.

**6.** The method according to claim 3,
wherein the measurement of the absorbance is carried out at a main wavelength in a range from 650 to 800 nm and at a sub-wavelength that is greater than the main wavelength and in a range from 730 to 900 nm.

**7.** The method according to claim 1,
wherein the surfactant is at least one surfactant selected from the group consisting of nonionic surfactants, alkyl sulfate, and high-molecular compounds.

**8.** The method according to claim 7,
wherein the nonionic surfactants are polyoxyethylene ethers in which a polyoxyethylene chain and a hydrocarbon chain are linked with each other by ether linkage.

**9.** The method according to claim 8,
wherein the polyoxyethylene chain has a weight-average degree of polymerization in a range from 8 to 23 and the hydrocarbon has a carbon number in a range from 8 to 18.

**10.** The method according to claim 8,
wherein the hydrocarbon chain is composed of at lease one of an alkyl group and an alkyl phenyl group

**11.** The method according to claim 8,
wherein the hydrocarbon chain has a branched chain.

**12.** The method according to claim 7,
wherein the high-molecular compounds is at least one compound selected from the group consisting of water-soluble gelatinum, pullulan, polyethylene glycol, and polyvinyl pyrrolidone.

**13.** The method according to claim 1,
wherein the surfactant is added to the sample so that a content of the surfactant per milliliter of the sample is in a range from 0.05 to 5 mol.

**14.** The method according to claim 1,
wherein the surfactant is added to the sample so that a content of the surfactant per mole of the tetrazolium compound is in a range from 0.2 to 15 mol.

**15.** The method according to claim 1,
wherein the tetrazolium compound contains benzene rings at a 2-position and 3-position on its tetrazole ring, and at least one of the benzene rings contains at least one functional group selected from the group consisting of a halogen group, a carboxy group, a nitro group, a hydroxy group, a sulfo group, a methoxy group, and an ethoxy group.

**16.** The method according to claim 1,
wherein the tetrazolium compound is 2-(4-iodophenyl)-3-(2,4-dinitrophenyl)-5-(2,4-disulfophenyl)-2H-tetrazolium salt.

**17.** The method according to claim 3,
wherein the oxidizing substance derived from the analyte is hydrogen peroxide, a color-developing substrate that develops color by oxidation is used as a color-developing substrate, and a redox reaction between the hydrogen peroxide and the color-developing substrate is caused by an oxidoreductase.

**18.** The method according to claim 1,
wherein the sample contains blood cells.

**19.** The method according to claim 1,
wherein the analyte is a glycated protein.

**20.** The method according to claim 1,
wherein the analyte is glycated hemoglobin.

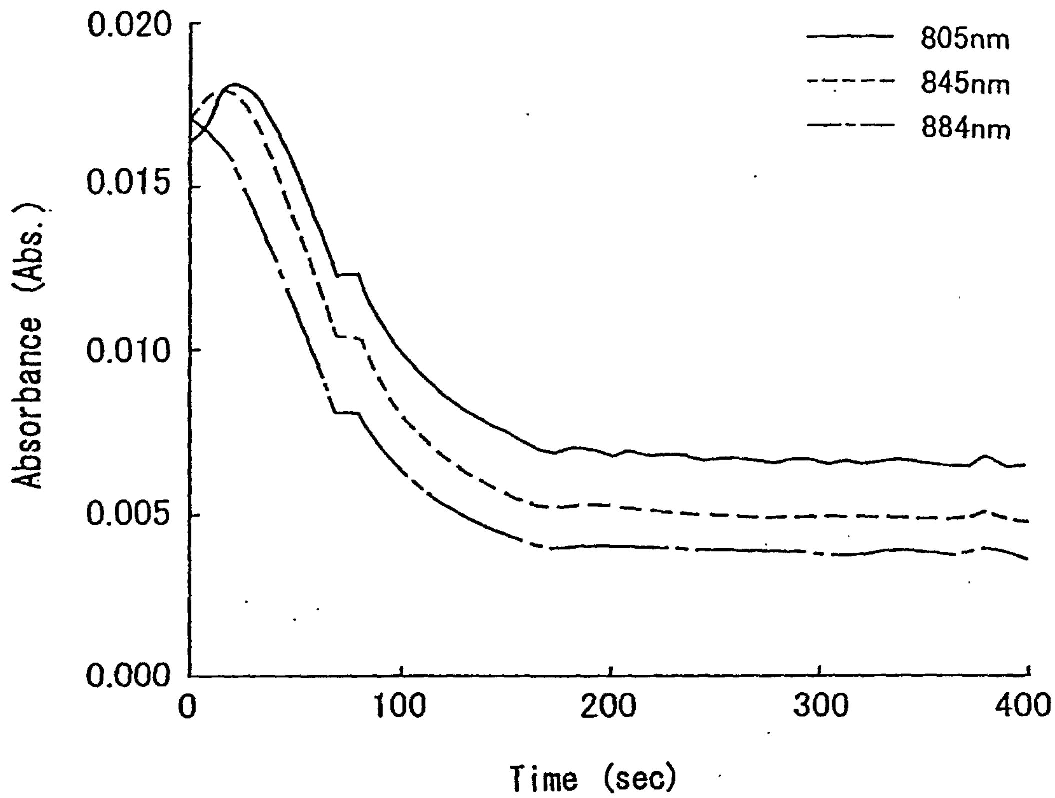
0.020
0.015
0.010
0.005
0.000
Absorbance (Abs.)
0
100
200
300
400
Time (sec)
805nm
845nm
884nm

FIG. 1

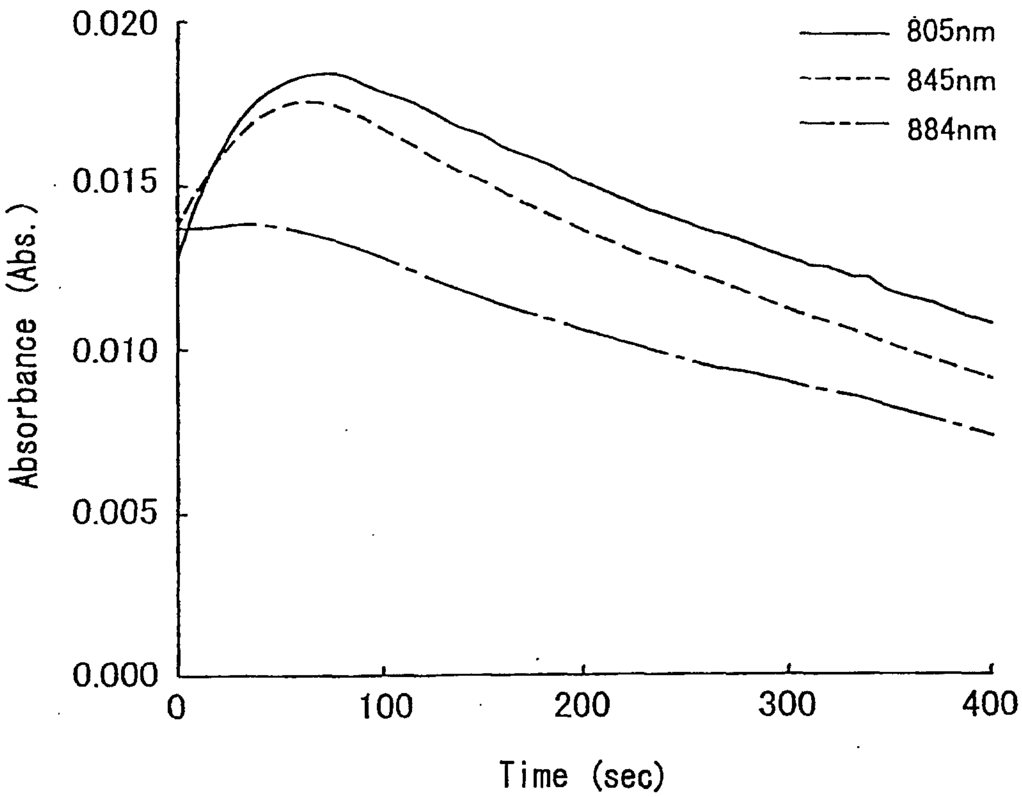
0.020
0.015
0.010
0.005
0.000
Absorbance (Abs.)
0
100
200
300
400
Time (sec)
805nm
845nm
884nm

FIG. 2

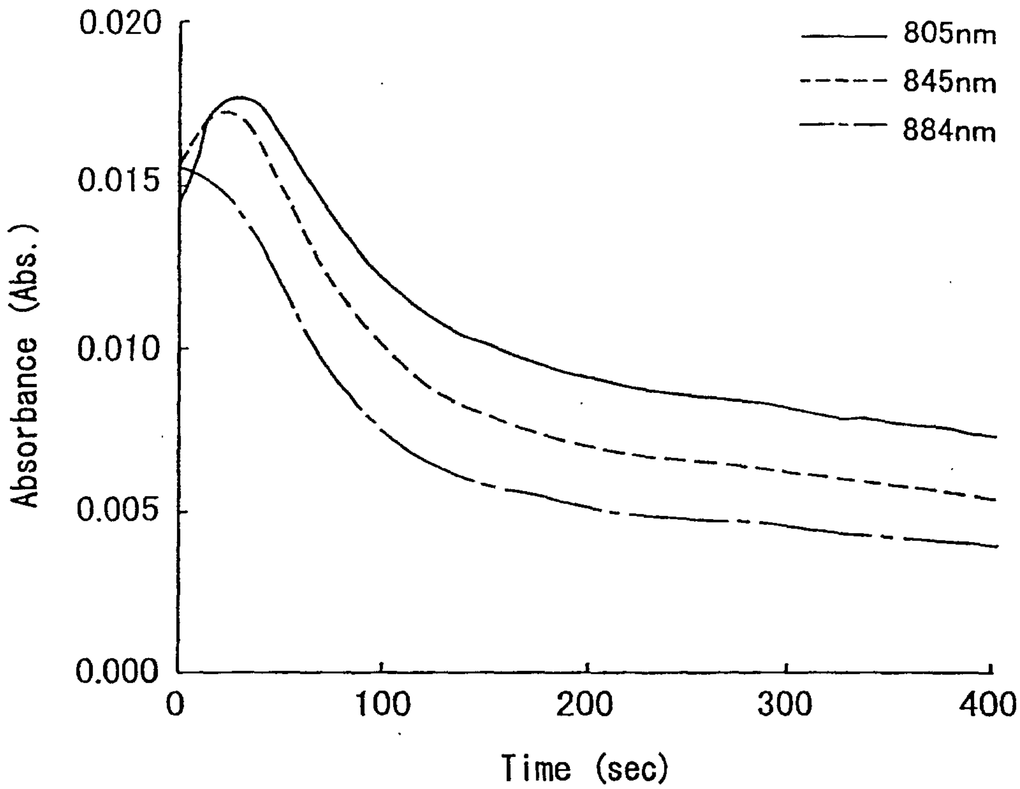

0.020
0.015
0.010
0.005
0.000
Absorbance (Abs.)
0
100
200
300
400
Time (sec)
805nm
845nm
884nm


FIG. 3

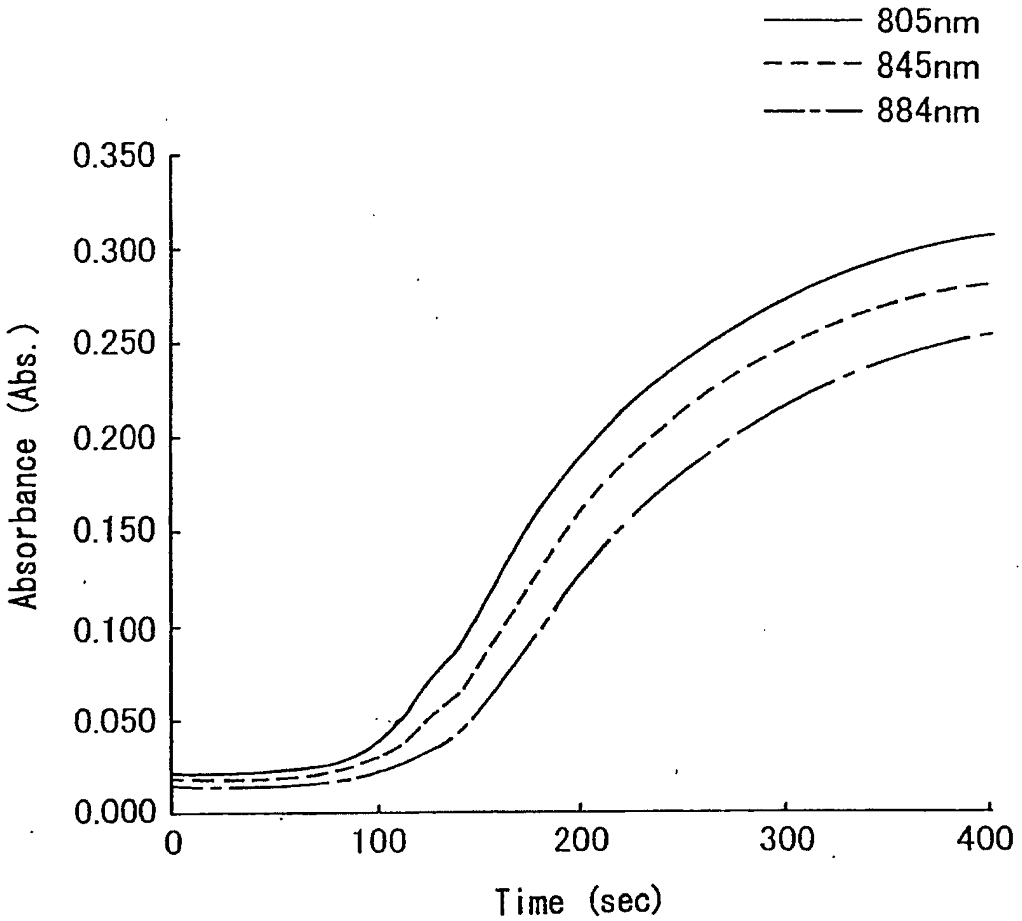
805nm
845nm
884nm
0.350
0.300
0.250
0.200
0.150
0.100
0.050
0.000
Absorbance (Abs.)
0
100
200
300
400
Time (sec)

FIG. 4

## INTERNATIONAL SEARCH REPORT

International application No.
PCT/JP01/08484

A. CLASSIFICATION OF SUBJECT MATTER
Int.Cl$^7$ G01N33/72, G01N21/78

According to International Patent Classification (IPC) or to both national classification and IPC

B. FIELDS SEARCHED

Minimum documentation searched (classification system followed by classification symbols)
Int.Cl$^7$ G01N33/48-33/98, G01N21/75-21/83

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2001 |
| Kokai Jitsuyo Shinan Koho | 1971-2001 | Jitsuyo Shinan Toroku Koho | 1996-2001 |

Electronic data base consulted during the international search (name of data base and, where practicable, search terms used)
JICST FILE (tetrazorium*surfactant)

C. DOCUMENTS CONSIDERED TO BE RELEVANT

| Category* | Citation of document, with indication, where appropriate, of the relevant passages | Relevant to claim No. |
|---|---|---|
| X | JP 2000-210100 A (KDK CORPORATION), 02 August, 2000 (02.08.00), Column 7, line 45 to Column 12, line 38, & EP 1002874 A2 | 1-20 |
| Y | JP 09-329598 A (KDK CORPORATION), 22 December, 1997 (22.12.97), Column 3, line 45 to Column 7, line 49, & EP 811844 A1 & US 5955027 A | 1-20 |
| Y | JP 2000-93197 A (Hoometto K.K.), 04 April, 2000 (04.04.00), Column 7, line 24 to Column 12, line 23, (Family: none) | 1-20 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>11 December, 2001 (11.12.01) | Date of mailing of the international search report<br>25 December, 2001 (25.12.01) |
| Name and mailing address of the ISA/<br>Japanese Patent Office | Authorized officer |
| Facsimile No. | Telephone No. |

Form PCT/ISA/210 (second sheet) (July 1992)